# EUROPEAN PATENT APPLICATION

(11) **EP 4 513 506 A1**
(43) Date of publication of application: **26.02.2025**
(21) Application number: 24194965.0
(22) Date of filing: 16.08.2024
(51) Int. Cl.: G16H 40/40, G16H 40/63

(54) **METHODS AND SYSTEMS FOR DYNAMICALLY LOADING ALGORITHMS BASED ON SURGERY INFORMATION**

(30) Priority: 18.08.2023 US 202363520588 P
(71) Applicant: Stryker Corporation, Portage, Michigan 49002 (US)
(72) Inventor: SUBRAMANIAN, Rohit, Portage, 49002 (US); PARAMASIVAN, Ramanan, Portage, 49002 (US); HUNTER, Brandon B., Portage, 49002 (US)
(74) Representative: V.O.

(57) **Abstract**

The present disclosure relates to dynamically providing machine learning models to a target device for a surgical procedure. An exemplary method can comprise: identifying a user profile associated with the surgical procedure; determining, based on the identified user profile, a plurality of candidate procedures; obtaining data from one or more devices in a surgical environment associated with the surgical procedure; selecting at least one procedure from the plurality of candidate procedures based on: the data obtained from the one or more devices, and one or more weights associated with the data obtained from the one or more devices; identifying a machine learning model based on the selected at least one procedure; and providing the identified machine learning model to the target device for execution.

## Description

### CROSS-REFERENCE TO RELATED APPLICATION

This application claims the benefit of U.S. Provisional Application 63/520,588 filed on August 18, 2023, the entire content of which is incorporated herein by reference for all purposes.

### FIELD

The present disclosure relates to dynamically loading algorithms (e.g., machine learning models) onto a target device based on surgery information.

### BACKGROUND

Increasingly, a variety of machine learning models are used to aid surgical procedures. For example, during cauterization, a smoke-removal machine learning model can be applied to intraoperative video data to enhance visualization of the video data. As another example, during a laparoscopic cholecystectomy procedure, a gallbladder Y junction detection model can be applied to help the surgeon identify and locate the clinical structures of interest to improve accuracy and safety. The exact machine learning models needed for a given surgical procedure may vary based on the surgeon, the procedure type, etc.

Currently, typically systems and devices used in a surgical environment (e.g., operating room) do not provide enough storage capacity to store an entire suite of machine learning models potentially applicable for all of the various surgical procedures. For example, many existing FPGA devices cannot accommodate one bitstream that encompasses a large number of machine learning models to be permanently loaded onto the devices. Further, FPGA devices with a larger capacity may be prohibitively expensive to acquire and maintain.

### SUMMARY

Disclosed herein are exemplary devices, apparatuses, systems, methods, and non-transitory storage media for dynamically providing machine learning models to a target device for a surgical procedure. Examples of the present disclosure can automatically identify a surgical procedure based on a variety of information (e.g., user profiles, device mode data, connected devices, contextual data, corresponding weights, etc.) as described herein. Based on the identified surgical procedure, one or more machine learning models can be identified and retrieved from a central location (e.g., local storage devices, remote servers) and dynamically loaded into an existing video pipeline, optionally along with any related processing blocks. An exemplary system can identify a user profile associated with the surgical procedure and determine, based on the identified user profile, a plurality of candidate procedures. The system can also obtain data from one or more devices in a surgical environment associated with the surgical procedure and select at least one procedure from the plurality of candidate procedures based on: the data obtained from the one or more devices, and one or more weights associated with the data obtained from the one or more devices. The system can then identify a machine learning model based on the selected at least one procedure and provide the identified machine learning model to the target device for execution.

If the target device is a programmable device such as an FPGA device, the system can provide a bitstream associated with the identified machine learning model to the programmable device. A bitstream comprises a file that contains the configuration information for an FPGA, such as the hardware logic, routing, and initial values for the registers and the on-chip memory. In some examples, the identified machine learning model, along with any related video processing algorithms, can be converted into one or more FPGA bitstreams and deployed onto the FPGA device. The related video processing algorithms may include, for example, dynamic range enhancement of image frames, overlay generation, smoke detection and removal, and the like. By streaming the bitstream to the FPGA's configuration port, the FPGA can be programmed to perform the intended task of the machine learning model.

In some examples, the system can provide the identified machine learning model to the target device for execution via a partial reconfiguration mechanism. Partial reconfiguration allows one or more portions of the FPGA device to be reconfigured (i.e., reprogrammed) while the rest of the FPGA device continues to operate without interruption. Accordingly, partial reconfiguration allows dynamic modification of certain sections of the FPGA device's configuration, rather than reprogramming the entire FPGA device.

In some examples, during the partial reconfiguration process, the system selects or identifies the one or more portions of the FPGA device to be reconfigured and loads the above-described bitstream associated with the identified machine learning model into the one or more portions while the rest of the FPGA device remains operational. The mechanism allows the FPGA device to update, modify, and/or remove its machine learning capabilities frequently, while other portions of the FPGA device remain operational.

In some examples, when the machine learning model is no longer needed (e.g., after the surgical procedure, after a particular stage of the surgical procedure, and the like), the programmable device can be reprogrammed using a default bitstream, thus removing the machine learning model from the programmable device. Alternatively, the programmable device can be reprogrammed via partial reconfiguration using a new bitstream associated with a different machine learning model, thus reprogramming portion(s) of the programmable device while the rest of the programmable device remains operational. If the target device is a processing device (e.g., containing a processor such as a central processing unit (CPU) or a graphics processing unit (GPU)), the system may provide a set of executable instructions (e.g., one or more programs) associated with the identified machine learning model to the processing device. The processing device stores the executable instructions in a memory unit and executes the instructions to perform the intended task of the machine learning model. When the machine learning model is no longer needed (e.g., after the surgical procedure, after a particular stage of the surgical procedure, and the like), the set of executable instructions may be deleted or overwritten from the memory unit.

Examples of the present disclosure provide numerous technical advantages. They allow a device to have access to multiple machine learning models for intraoperative use without requiring the storage capacity to store one or more of the multiple machine learning models. This is especially beneficial for devices used in a typical surgical environment, which are often resource-constrained and would not otherwise have the storage capacity to store one or more of the machine learning models. After a machine learning model is loaded and used by a device, the machine learning model may be removed when it is not in use to free up storage space, thus improving the processing speed, the storage capacity, and the performance of the device. Examples of the present disclosure can also eliminate the need for acquiring, configuring, and maintaining devices having larger storage capacities for the express purpose of running machine learning models. Further, the machine learning models can be centrally maintained and managed (e.g., by an administrator, medical staff, etc.) in an efficient manner and can be retrained to improve their performance. As described herein, the system may further intelligently select a target device based on a storage capacity and/or status of the target device and may dynamically determine an appropriate version of the machine learning model based on the selected target device. Accordingly, examples of the present disclosure can result in a system that is more flexible, efficient, robust, and economical than conventional systems that require larger storage capacities to accommodate storage of machine learning models.

According to an aspect is provided a method for dynamically providing machine learning models to a target device for a surgical procedure. The method comprises determining a plurality of candidate procedures. The method may comprise identifying a user profile associated with the surgical procedure, and determining, based on the identified user profile, a plurality of candidate procedures. The method may comprise obtaining data from one or more devices in a surgical environment associated with the surgical procedure. The method comprises selecting at least one procedure from the plurality of candidate procedures, e.g. based on the data obtained from the one or more devices, and one or more weights associated with the data obtained from the one or more devices. The method comprises identifying a machine learning model based on the selected at least one procedure. The method may comprise providing the identified machine learning model to the target device for execution.

Optionally, the identified user profile comprises: an identity of a user, one or more specialties of the user, one or more device settings, one or more procedure types, or any combination thereof.

Optionally, the user profile is identified based on: a user input indicative of the identity of the user, audio data associated with the surgical environment, image data associated with the surgical environment, location information associated with the user, or any combination thereof.

Optionally, the one or more devices comprise at least one imager, and the data obtained from the one or more devices comprises device mode data associated with the at least one imager.

Optionally, the device mode data comprises: one or more camera specialty settings, one or more image modes, or any combination thereof.

Optionally, the one or more devices comprise at least one surgical instrument, and the data obtained from the one or more devices is indicative of whether the at least one surgical instrument is active.

Optionally, the one or more devices comprise at least one sensor, and the data obtained from the one or more devices comprises contextual information obtained by the sensor.

Optionally, the at least one sensor comprises an image sensor, an audio sensor, or any combination thereof.

Optionally, the one or more weights are determined based on a time and/or a time duration associated with at least a portion of the data from the one or more devices.

Optionally, the one or more weights are determined based on device type data associated with at least one of the one or more devices.

Optionally, the target device is selected from a plurality of candidate target devices based on a state of the target device and a storage capacity of the target device.

Optionally, the plurality of candidate devices comprises: a display, a camera, a programmable device, a processing device, or any combination thereof.

Optionally, providing the identified machine learning model to the target device for execution comprises: selecting a version of the identified machine learning model from a plurality of versions of the identified machine learning model; and providing the selected version of the identified machine learning model to the target device.

Optionally, the version of the identified machine learning model is selected based on a device type of the target device.

Optionally, the version of the identified machine learning model is selected based on medical history of one or more patients.

Optionally, the target device comprises a programmable device, and wherein providing the identified machine learning model to the target device comprises: providing a bitstream associated with the identified machine learning model to the programmable device. In some examples, the target device comprises a processing device, and wherein providing the identified machine learning model to the target device comprises: providing a set of executable instructions associated with the identified machine learning model to the processing device.

According to an aspect is provided a system for dynamically providing machine learning models to a target device for a surgical procedure. The system comprises one or more processors; one or more memories; and one or more programs, wherein the one or more programs are stored in the one or more memories and configured to be executed by the one or more processors. The one or more programs include instructions for determining a plurality of candidate procedures. The one or more programs may include instructions for identifying a user profile associated with the surgical procedure; and determining, based on the identified user profile, a plurality of candidate procedures. The one or more programs may include instructions for obtaining data from one or more devices in a surgical environment associated with the surgical procedure. The one or more programs include instructions for selecting at least one procedure from the plurality of candidate procedures, e.g. based on the data obtained from the one or more devices, and one or more weights associated with the data obtained from the one or more devices. The one or more programs include instructions for identifying a machine learning model based on the selected at least one procedure. The one or more programs may include instructions for providing the identified machine learning model to the target device for execution.

Optionally, the identified user profile comprises: an identity of a user, one or more specialties of the user, one or more device settings, one or more procedure types, or any combination thereof.

Optionally, the user profile is identified based on: a user input indicative of the identity of the user, audio data associated with the surgical environment, image data associated with the surgical environment, location information associated with the user, or any combination thereof.

Optionally, the one or more devices comprise at least one imager, and the data obtained from the one or more devices comprises device mode data associated with the at least one imager.

Optionally, the device mode data comprises: one or more camera specialty settings, one or more image modes, or any combination thereof.

Optionally, the one or more devices comprise at least one surgical instrument, and the data obtained from the one or more devices is indicative of whether the at least one surgical instrument is active.

Optionally, the one or more devices comprise at least one sensor, and the data obtained from the one or more devices comprises contextual information obtained by the sensor.

Optionally, the at least one sensor comprises an image sensor, an audio sensor, or any combination thereof.

Optionally, the one or more weights are determined based on a time and/or a time duration associated with at least a portion of the data from the one or more devices.

Optionally, the one or more weights are determined based on device type data associated with at least one of the one or more devices.

Optionally, the target device is selected from a plurality of candidate target devices based on a state of the target device and a storage capacity of the target device.

Optionally, the plurality of candidate devices comprises: a display, a camera, a programmable device, a processing device, or any combination thereof.

Optionally, providing the identified machine learning model to the target device for execution comprises: selecting a version of the identified machine learning model from a plurality of versions of the identified machine learning model; and providing the selected version of the identified machine learning model to the target device.

Optionally, the version of the identified machine learning model is selected based on a device type of the target device.

Optionally, the version of the identified machine learning model is selected based on medical history of one or more patients.

Optionally, the target device comprises a programmable device, and wherein providing the identified machine learning model to the target device comprises: providing a bitstream associated with the identified machine learning model to the programmable device.

Optionally, the target device comprises a processing device, and wherein providing the identified machine learning model to the target device comprises: providing a set of executable instructions associated with the identified machine learning model to the processing device.

According to an aspect is provided an exemplary non-transitory computer-readable storage medium stores one or more programs, the one or more programs comprising instructions, which when executed by one or more processors of an electronic device, cause the electronic device to perform any of the methods described herein. According to an aspect is provided a computer program product, comprising instructions, which when executed by one or more processors of an electronic device, cause the electronic device to perform any of the methods described herein.

It will be appreciated that any of the variations, aspects, features and options described in view of the systems apply equally to the methods and vice versa. It will also be clear that any one or more of the above variations, aspects, features and options can be combined.

### BRIEF DESCRIPTION OF THE FIGURES

The invention will now be described, by way of example only, with reference to the accompanying drawings, in which:
FIG. 1 illustrates an exemplary operating room hub controller system, in accordance with some examples.
FIG. 2 illustrates an exemplary process for dynamically providing machine learning models to a target device for a surgical procedure, in accordance with some examples.
FIG. 3 illustrates an exemplary electronic device, in accordance with some examples.

### DETAILED DESCRIPTION

Reference will now be made in detail to implementations and examples of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Disclosed herein are exemplary devices, apparatuses, systems, methods, and non-transitory storage media for dynamically providing machine learning models to a target device for a surgical procedure. Examples of the present disclosure can automatically identify a surgical procedure based on a variety of information (e.g., user profiles, device mode data, connected devices, contextual data, corresponding weights, etc.) as described herein. Based on the identified surgical procedure, one or more machine learning models can be identified and retrieved from a central location (e.g., local storage devices, remote servers) and dynamically loaded into an existing video pipeline, optionally along with any related processing blocks. An exemplary system can identify a user profile associated with the surgical procedure and determine, based on the identified user profile, a plurality of candidate procedures. The system can also obtain data from one or more devices in a surgical environment associated with the surgical procedure and select at least one procedure from the plurality of candidate procedures based on: the data obtained from the one or more devices, and one or more weights associated with the data obtained from the one or more devices. The system can then identify a machine learning model based on the selected at least one procedure and provide the identified machine learning model to the target device for execution. A user profile may include a profile of an individual or a group of individuals associated with the surgical procedure, as described herein. In some examples, the user profile can include a surgeon profile, a medical staff profile, an administrator profile, or any combination thereof.

If the target device is a programmable device such as an FPGA device, the system can provide a bitstream associated with the identified machine learning model to the programmable device, which is programmed using the bitstream to perform the intended task of the machine learning model. In some examples, the FPGA device can be updated via partial reconfiguration as described above. When the machine learning model is no longer needed (e.g., after the surgical procedure, after a particular stage of the surgical procedure), the programmable device can be reprogrammed using a default or alternative bitstream, thus removing the machine learning model from the programmable device and/or replacing the machine learning model with one or more new machine learning models. If the target device is a processing device (e.g., containing a processor such as a central processing unit (CPU) or a graphics processing unit (GPU)), the system may provide a set of executable instructions (e.g., one or more programs) associated with the identified machine learning model to the processing device. The processing device stores the executable instructions in a memory unit and executes the instructions to perform the intended task of the machine learning model. When the machine learning model is no longer needed (e.g., after the surgical procedure, after a particular stage of the surgical procedure), the set of executable instructions may be deleted or overwritten from the memory unit.

Examples of the present disclosure provide numerous technical advantages. They allow a device to have access to multiple machine learning models for intraoperative use without having the storage capacity to store all of these machine learning models. This is especially beneficial for devices used in a typical surgical environment, which are often resource-constrained and would not otherwise have the storage capacity to store all the machine learning models. After a machine learning model is loaded and used by a device, the machine learning model may be removed when it is not in use to free up storage space, thus improving the processing speed, the storage capacity, and the performance of the device. Examples of the present disclosure can also eliminate the need for acquiring, configuring, and maintaining devices with larger storage capacity just for the purpose of running machine learning models. Further, the machine learning models can be centrally maintained and managed in an efficient manner and can be retrained to improve its performance to benefit any target device. As described herein, the system may further intelligently select a target device based on its storage capacity and status and dynamically determine an appropriate version of the machine learning model based on the selected target device. Accordingly, examples of the present disclosure can result in a system that is more flexible, efficient, robust, and economical.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

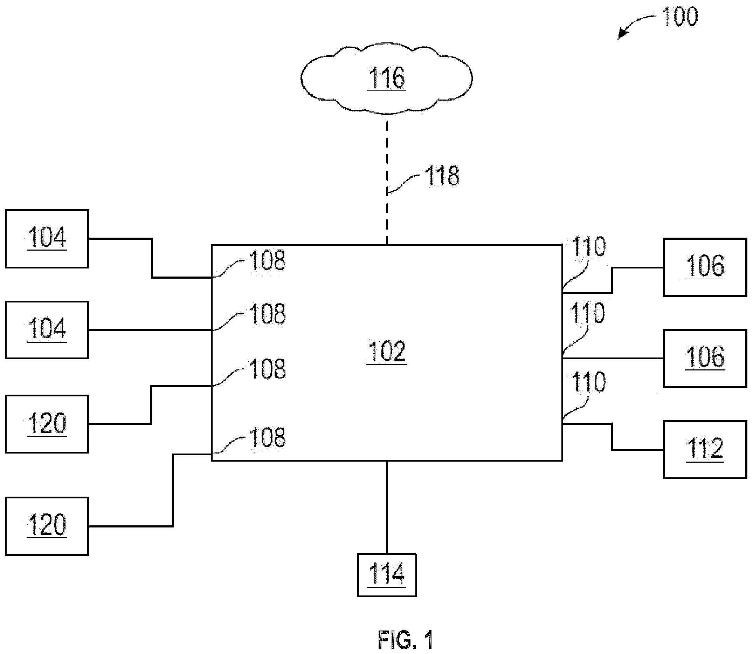

FIG. 1 illustrates a system 100 for generating and displaying medical imaging data during medical imaging sessions. System 100 includes a medical data processing hub 102 that processes data received from one or more imaging modalities 104 to generate one or more display feeds for displaying enhanced medical imaging on one or more displays 106. The one or more imaging modalities 104 may generate image data associated with treatment of a patient. The image data can be images or videos generated during treatment of the patient in support of one or more medical procedures, such as video captured by an endoscopic camera during an endoscopic procedure on a patient. Examples of medical imaging modalities include, without limitation, endoscopic systems, open field imaging systems, x-ray systems such as intraoperative c-arm systems, computer tomography systems, ultrasound systems, magnetic resonance imaging systems, and nuclear medicine systems.

In some examples, the medical data processing hub 102 may receive data from one or more non-imaging devices 120 that may be used in connection with (e.g., during) a medical imaging session and that may provide information that may be relevant for display during a medical imaging session. Non-limiting examples of non-imaging devices 120 include insufflators, illumination controllers, and voice control systems.

The medical data processing hub 102 may receive image data from the one or more imaging modalities 104 through one or more input ports 108. The medical data processing hub 102 generates one or more display feeds using received imaging data and transmits the one or more display feeds to one or more displays 106 via one or more output ports 110. For example, the medical data processing hub 102 may generate a display feed that includes enhanced imaging of tissue of a patient based on imaging generated by one or more imaging modalities 104, and the enhanced imaging may be displayed on one or more of the displays 106 to assist a user (e.g., a surgeon, a nurse, other medical personnel) during treatment of the patient. The medical data processing hub 102 may also transmit display feeds to one or more recording devices 112 for recording enhanced imaging for later retrieval. Input ports 108 and output ports 110 may be any suitable types of data transmission ports, such as DVI ports, HDMI ports, RS232 ports, IP ports, and the like.

The medical data processing hub 102 may be connected to one or more networks 116 via one or more network connections 118. The one or more networks 116 may be a local network such as a hospital information system or may be a wider network such as a wide area network or the internet. A network connection 118 can be a wired connection, such as an Ethernet connection, or a wireless network connection, such as a Wi-Fi connection. In some examples, the medical data processing hub 102 may access the one or more networks 116 to retrieve configuration data stored at a network location for configuring the medical data processing hub 102 for an imaging session, and/or may access the one or more networks to receive updated software and/or updated hardware files for processing imaging data.

One or more user interfaces 114 may be connected to the medical data processing hub 102 for a user to provide input to the medical data processing hub 102. The user may input data related to configuring the medical data processing hub 102 for an imaging session. User input can include, for example, selection of a practitioner profile (e.g., a user profile as described herein) associated with an upcoming imaging session, selection of a type of imaging session or type of procedure to be performed during an imaging session, or any other relevant information. The one or more user interfaces 114 may include a tablet, a keyboard, a mouse, a voice control system, a keypad, a touchscreen, or any combination thereof. The user interface 114 may have a wired or wireless connection. The input may be provided locally or remotely such as off-site from the medical facility (e.g., by an administrator or third party).

The medical data processing hub 102 may further comprise one or more FPGAs, one or more CPUs, one or more GPUs, or any combination thereof. As described in detail below, examples of the present disclosure can automatically identify a surgical procedure based on a variety of information (e.g., user profiles, device mode data, connected devices, contextual data, corresponding weights, etc.) as described herein. Based at least in part on the identified surgical procedure, one or more machine learning models can be identified and retrieved by the medical data processing hub 102 from a central location (e.g., local storage devices, remote servers) and dynamically loaded onto the medical data processing hub 102. The machine learning models described herein may be stored on a local network (e.g., of the medical facility) and/or stored on the cloud (e.g., managed by a third party). In some examples, a third party can manage the machine learning models on a subscription basis. For example, a medical facility can subscribe for the use of the machine learning models on-demand.

If the medical data processing hub 102 includes a programmable device such as an FPGA device, a bitstream associated with the identified machine learning model can be provided to the programmable device of the medical data processing hub 102, which is programmed using the bitstream to perform the intended task of the machine learning model (e.g., via partial reconfiguration as described above). When the machine learning model is no longer needed (e.g., after the surgical procedure, after a particular stage of the surgical procedure), the programmable device of the system 100 can be reprogrammed using a default bitstream or an alternative bitstream, thus removing the machine learning model from the programmable device of the medical data processing hub 102 and/or replacing the machine learning model with one or more new machine learning models. If the medical data processing hub 102 comprises a CPU and/or a GPU, a set of executable instructions (e.g., one or more programs) associated with the identified machine learning model can be provided to the medical data processing hub 102. The CPU and/or GPU of the medical data processing hub 102 can execute the instructions to perform the intended task of the machine learning model. When the machine learning model is no longer needed (e.g., after the surgical procedure, after a particular stage of the surgical procedure), the set of executable instructions may be deleted from the medical data processing hub 102.

It should be appreciated that examples of the present disclosure can be used to load machine learning model(s) onto other types of target devices associated with a surgical environment or the system 100, such as displays (e.g., the one or more displays 106) and medical instruments (e.g., the one or more non-imaging devices 120).

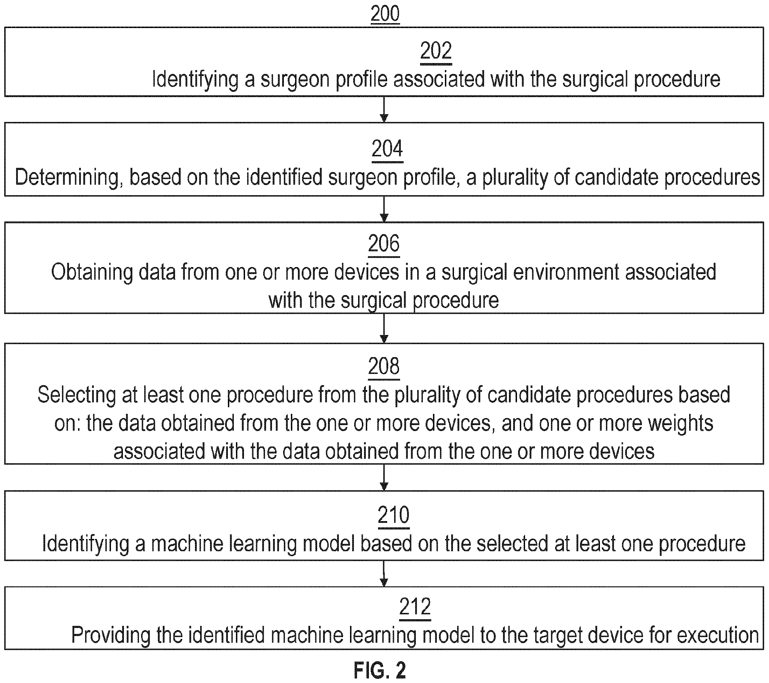

FIG. 2 illustrates an exemplary process 200 for dynamically providing machine learning models to a target device, such as the medical data processing hub 102 of the system 100, the one or more displays 106 of the system 100, and the one or more non-imaging devices 120 of the system 100, for a surgical procedure. Process 200 is performed, for example, using one or more electronic devices implementing a software platform, which may or may not be part of the system 100 in FIG. 1. Process 200 can be performed using a client-server system, and the blocks of process 200 are divided up in any manner between the server and a client device. Alternatively, the blocks of process 200 are divided up between the server and multiple client devices. Alternatively, process 200 is performed using only a client device or only multiple client devices. In process 200, some blocks are, optionally, combined, the order of some blocks is, optionally, changed, and some blocks are, optionally, omitted. Additional steps may be performed in combination with the process 200. Accordingly, the operations as illustrated (and described in greater detail below) are exemplary by nature and, as such, should not be viewed as limiting.

At block 202, an exemplary system (e.g., one or more electronic devices) identifies a user profile associated with the surgical procedure. The identified user profile can comprise information related to one or more users (e.g., an individual user, a group of users). For example, the identified user profile may comprise an identity of the user (e.g., a surgeon, a medical staff member, or an administrator), one or more specialties of the user, one or more device settings specific to the user, one or more procedure types specific to the user, or any combination thereof. The one or more device settings may be based on settings used by the user in previous surgical procedures, preferences or configurations indicated by the user, or any combination thereof. The one or more procedure types may include procedure types that the user can perform (e.g., based on their specialty, based on their qualifications) and/or has performed in previous surgical procedures.

The system may identify the user profile based on user input indicative of the identity of the user, audio data associated with the surgical environment, image data associated with the surgical environment, location information associated with the user, or any combination thereof. Specifically, a user (e.g., the surgeon) may provide a user input indicative of his or her identity as a part of the preoperative or intraoperative procedure, such as logging into one or more devices (e.g., the medical data processing hub 102 of FIG. 1) using his or her credentials, selecting his or her profile from a plurality of user profiles on one or more devices (e.g., the medical data processing hub 102), scanning a badge, etc. Based on the user input, the system can identify the user and retrieve the corresponding user profile. As another example, the system may receive audio data (e.g., utterances, conversations, surgical checklist discussions) and/or image data (e.g., photographs, videos) associated with the surgical environment (e.g., an operating room). The system can perform speech recognition, semantic analysis, and/or biometric analysis of the audio data to identify the user and retrieve the corresponding user profile. The system can also perform facial recognition of the image data to identify the people in the operating room, including the user, and retrieve the corresponding user profile. As another example, the system may receive location information such as GPS locations of various users (e.g., obtained from devices of the surgeons) and compare the location information with the location information of the surgical environment to determine which user is in the surgical environment.

At block 204, the system determines, based on the identified user profile, a plurality of candidate procedures. The candidate procedures can include procedures that the user is qualified to perform and/or has performed in the past. The system can directly retrieve the candidate procedures from the user profile. Alternatively or additionally, the system can derive the candidate procedures based on the information in the user profile. For example, based on the user's specialty, the system can retrieve one or more procedures that any user in the specific specialty is qualified to perform. As another example, based on the user's identity, the system can retrieve one or more procedures that the user has performed in the past from one or more databases.

In some examples, in addition to or instead of relying on the user profile, the system may determine the plurality of candidate procedures based on one or more user inputs indicative of the type of procedure to be performed. As described above with reference to FIG. 1, a user may input data related to configuring the medical data processing hub 102 for an imaging session, which may include selection of a type of imaging session or a type of procedure to be performed during an imaging session. Accordingly, such information may be used by the system to determine the plurality of candidate procedures in block 204 in FIG. 2.

At block 206, the system obtains data from one or more devices in the surgical environment associated with the surgical procedure. The data can include device mode data, data related to connected surgical instruments, contextual data, or any combination thereof, as described herein.

In some examples, the one or more devices in the surgical environment may comprise at least one imager (e.g., the one or more imaging modalities 104 of system 100 in FIG. 1), and the data obtained from the one or more devices may comprise device mode data associated with the at least one imager. Specifically, the device mode data may comprise one or more camera specialty settings, one or more image modes (e.g., image mode selected for a given camera specialty), or any combination thereof. The camera specialty settings may include settings associated with arthroscopy, cystoscopy, otorhinolaryngology, flexi-scope, laparoscopy, laser, microscope, spine surgery, or the like. The image modes may include overlay, contrast, endoscopic near infrared visualization (ENV), color segmented fluorescence (CSF), chemical shift imaging (CSI), or the like.

In some examples, the one or more devices comprise at least one surgical instrument or medical device (e.g., the one or more non-imaging devices 120 of system 100 in FIG. 1), and the data obtained from the one or more devices may comprise information related to the surgical instrument or medical device. The data may be indicative of which surgical instrument or medical device in the operating room is active (e.g., turned on, connected to the medical data processing hub 102 in FIG. 1, connected to a pump/shaver, in operation). The data may also include settings of the active surgical instrument or medical device. For example, before or during a surgical procedure, a user (e.g., a surgeon, a nurse, other medical personnel) may activate (e.g., turn on, connect to the medical data processing hub 102 in FIG. 1) different devices depending on the surgical procedure to be performed. Further, the user may configure a device differently depending on the surgical procedure to be performed. For example, for a gallbladder removal procedure, the user may load the gallbladder preset on a device. As another example, for a procedure on a child, the user may put a surgical instrument into a pediatric mode. As another example, different light sources may be activated and the active light source may be configured differently depending on the surgical procedure. For example, certain surgical procedures may utilize lighted ureteral stents, which can be fed into a patient's ureter and then illuminated for ureter imaging. The system may be able to detect when the stents are connected to the illumination source, which can be used to identify machine-learning model(s) according to the techniques described herein. Additional examples include insufflator pressure settings, one or more pump settings and connection information, etc. Thus, the activation of specific surgical instrument(s) or medical device(s), as well as their settings, can be indicative of the specific surgical procedure being performed or to be performed.

In some examples, the one or more devices comprise at least one sensor, and the data obtained from the one or more devices comprises contextual information obtained by the sensor. The at least one sensor may comprise an image sensor, an audio sensor, or any combination thereof. The image sensor(s) can be located in the operating room (e.g., pan-tilt-zoom or PTZ cameras) and/or attached as a part of a medical device (e.g., endoscopic cameras). For example, a PTZ camera may be used to obtain images of various objects and people in the operating room and the images can be analyzed (e.g., using machine learning models such as object detection models) to determine which surgical procedure is being, or will be, performed and optionally a stage of the surgical procedure, which can be identified based on the medical devices used, the people in the room, activities in the room, etc. As another example, an endoscopic camera can be used to obtain images of the inside of the patient body and the images can be analyzed (e.g., using machine learning models such as object detection models) to determine which surgical procedure is being, will be, performed and optionally a stage of the surgical procedure, which can be identified based on the tools attached to the endoscope, the objects in view of the endoscope (e.g., organs, blood, smoke, debris, bubbles), the activity in view of the endoscope (e.g., navigation, cauterization, incision), etc. The audio sensor(s) can be located in the operating room to obtain audio data (e.g., utterances, conversations, surgical checklist discussions). The system can perform speech recognition and semantic analysis of the audio data to identify which surgical procedure is being, will be, performed and optionally a stage of the surgical procedure.

At block 208, the system selects at least one procedure from the plurality of candidate procedures based on the data obtained from the one or more devices and one or more weights associated with the data obtained from the one or more devices. The one or more weights can be determined based on a time and/or a time duration associated with at least a portion of the data from the one or more devices. Data that is obtained more recently may be assigned a higher weight because it is more likely to provide the most up-to-date information. For example, if the system receives a first data indicative of a first procedure and a second data indicative of a second procedure different from the first procedure, the system may assign a higher weight to the second data if the second data is obtained later. As a result, the system may select the second procedure. The one or more weights can also be determined based on device type data associated with at least one of the one or more devices. For example, device mode data, device settings, and which devices are active (e.g., connected to the medical data processing hub 102 of FIG. 1) may be weighted higher than contextual data such as conversations obtained by the audio sensors in the operating room, as the former may provide more definitive and less ambiguous evidence of the surgical procedure.

At block 210, the system identifies a machine learning model based on the selected at least one procedure. As discussed above, different machine learning models may be trained to aid different procedures. Thus, based on the selected at least one procedure, the system can identify a machine learning model that is trained to aid the selected at least one procedure. As an example, for an arthroscopy procedure, the system may identify a blood detection algorithm. As another example, for a laparoscopic cholecystectomy procedure, the system may identify a gallbladder Y junction detection algorithm. Optionally, the selection of the procedure in block 208 and/or the identification of the machine learning model in block 210 can be associated with a confidence value (e.g., between 0 and 1). If the confidence value is below a predefined threshold (e.g., 0.75), the system may provide a prompt for user confirmation (e.g., "Would you like to use an algorithm for smoke removal?"). The confidence value can be determined based on a time and/or a time duration associated with the data used to identify the algorithm. Data that is obtained more recently may result in a higher confidence value because the data is more likely to provide the most up-to-date information. The confidence value can also be determined based on the type of data used to identify the algorithm. For example, device mode data, device settings, and which devices are active (e.g., connected to the medical data processing hub 102 of FIG. 1) may result in a higher confidence score than contextual data such as conversations obtained by the audio sensors in the operating room, as the former may provide more definitive and less ambiguous evidence of the surgical procedure.

At block 212, the system provides the identified machine learning model to the target device for execution. The identified machine learning model (or multiple versions of the machine learning model as discussed below) may be located in a local or remote storage device. The system may retrieve (e.g., retrieve from local memory, download) the identified machine learning model and transmit the machine learning model to the target device for execution. Optionally, the machine learning model can be updated (e.g., iteratively retrained using different training datasets) at the storage device. Accordingly, the system may retrieve the most recently trained version of the machine learning model to the target device for improved performance.

The target device may be a programmable device such as an FPGA device, and the system can provide a bitstream associated with the identified machine learning model to the programmable device. After the bitstream is provided to the programmable device, the programmable device is programmed using the bitstream to perform the intended task of the machine learning model (e.g., via partial reconfiguration). When the machine learning model is no longer needed (e.g., after the surgical procedure, after a stage of the surgical procedure), the programmable device can be reprogrammed using a default bitstream or an alternative bitstream, thus removing the machine learning model from the programmable device and/or replacing the machine learning model with one or more new machine learning models.

The target device may be a processing device (e.g., containing a processor such as a CPU or a GPU), and the system may provide a set of executable instructions (e.g., one or more programs) associated with the identified machine learning model to the processing device. The processing device stores the executable instructions in a memory unit and executes the instructions to perform the intended task of the machine learning model. When the machine learning model is no longer needed (e.g., after the surgical procedure, after a stage of the surgical procedure), the set of executable instructions may be deleted or overwritten from the memory unit.

In some examples, multiple target devices are available, including a display, a camera, a programmable device (e.g., FPGA), and/or a processing device (e.g., GPU, CPU), and the system may select the target device from a plurality of candidate target devices to provide the identified machine learning model to. The selection may be based on a state of the target device and a storage capacity of the target device. For example, the system may forego the selection of a candidate target device if the candidate target device is currently executing another process and thus has insufficient storage and/or processing capacity. In some examples, the system may access a ranked list of candidate target devices and select the first candidate target device on the list that has sufficient storage and processing capacity.

In some examples, a machine learning model may have multiple versions that are designed to be used on different types of target devices. For example, a machine learning model may have a bitstream version to be used on an FPGA and an executable code version to be used on a computer having a CPU. Accordingly, based on a device type of the target device, the system can select a version of the identified machine learning model from a plurality of versions of the identified machine learning model and provide the selected version of the identified machine learning model to the target device.

In some examples, a machine learning model may have multiple versions corresponding to different preexisting conditions. For example, a machine learning model for analyzing images of lungs may have a first version trained using images of smokers' lungs and a second version trained using images of non-smokers' lungs. Accordingly, based on a patient's medical history (e.g., chronic disease such as diabetes and high blood pressure, deformities, prior surgeries such as tumor removals), the system can select a version of the identified machine learning model from a plurality of versions of the identified machine learning model and provide the selected version of the identified machine learning model to the target device.

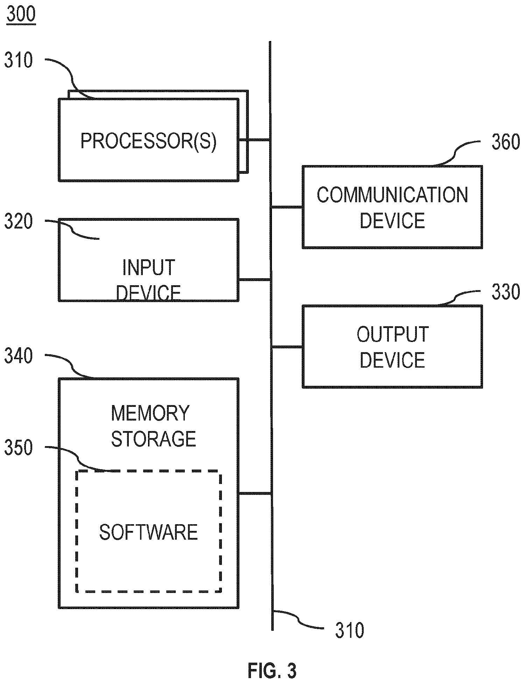

FIG. 3 illustrates an example of a computing device in accordance with one embodiment. The operations described above with reference to FIG. 2 are optionally implemented by components depicted in FIG. 3. Device 300 can be a host computer connected to a network. Device 300 can be a client computer or a server. As shown in FIG. 3, device 300 can be any suitable type of microprocessor-based device, such as a personal computer, workstation, server or handheld computing device (portable electronic device) such as a phone or tablet. The device can include, for example, one or more of processor 310, input device 320, output device 330, storage 340, and communication device 360. Input device 320 and output device 330 can generally correspond to those described above, and can either be connectable or integrated with the computer.

Input device 320 can be any suitable device that provides input, such as a touch screen, keyboard or keypad, mouse, or voice-recognition device. Output device 330 can be any suitable device that provides output, such as a touch screen, haptics device, or speaker.

Storage 340 can be any suitable device that provides storage, such as an electrical, magnetic or optical memory including a RAM, cache, hard drive, or removable storage disk. Communication device 360 can include any suitable device capable of transmitting and receiving signals over a network, such as a network interface chip or device. The components of the computer can be connected in any suitable manner, such as via a physical bus or wirelessly.

Software 350, which can be stored in storage 340 and executed by processor 310, can include, for example, the programming that embodies the functionality of the present disclosure (e.g., as embodied in the devices as described above).

Software 350 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 340, that can contain or store programming for use by or in connection with an instruction execution system, apparatus, or device.

Software 350 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 300 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T3 or T3 lines, cable networks, DSL, or telephone lines.

Device 300 can implement any operating system suitable for operating on the network. Software 350 can be written in any suitable programming language, such as C, C++, Java or Python. In various embodiments, application software embodying the functionality of the present disclosure can be deployed in different configurations, such as in a client/server arrangement or through a Web browser as a Web-based application or Web service, for example.

The foregoing description, for the purpose of explanation, has been described with reference to specific examples or aspects. However, the illustrative discussions above are not intended to be exhaustive or to limit the invention to the precise forms disclosed. For the purpose of clarity and a concise description, features are described herein as part of the same or separate variations; however, it will be appreciated that the scope of the disclosure includes variations having combinations of all or some of the features described. Many modifications and variations are possible in view of the above teachings. The variations were chosen and described in order to best explain the principles of the techniques and their practical applications. Others skilled in the art are thereby enabled to best utilize the techniques and various variations with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

For the purpose of clarity and a concise description, features are described herein as part of the same or separate examples; however, it will be appreciated that the scope of the disclosure includes examples having combinations of all or some of the features described.

## Claims

1. A method for dynamically providing machine learning models to a target device for a surgical procedure, comprising:
identifying a user profile associated with the surgical procedure;
determining, based on the identified user profile, a plurality of candidate procedures;
obtaining data from one or more devices in a surgical environment associated with the surgical procedure;
selecting at least one procedure from the plurality of candidate procedures based on:
the data obtained from the one or more devices, and
one or more weights associated with the data obtained from the one or more devices;
identifying a machine learning model based on the selected at least one procedure; and
providing the identified machine learning model to the target device for execution.

2. The method of claim 1, wherein the identified user profile comprises: an identity of a user, one or more specialties of the user, one or more device settings, one or more procedure types, or any combination thereof.

3. The method of claim 2, wherein the user profile is identified based on:
a user input indicative of the identity of the user,
audio data associated with the surgical environment,
image data associated with the surgical environment,
location information associated with the user,
or any combination thereof.

4. The method of any one of the preceding claims,
wherein the one or more devices comprise at least one imager, and
wherein the data obtained from the one or more devices comprises device mode data associated with the at least one imager.

5. The method of claim 4, wherein the device mode data comprises: one or more camera specialty settings, one or more image modes, or any combination thereof.

6. The method of any one of the preceding claims,
wherein the one or more devices comprise at least one surgical instrument, and
wherein the data obtained from the one or more devices is indicative of whether the at least one surgical instrument is active.

7. The method of any one of the preceding claims,
wherein the one or more devices comprise at least one sensor, and
wherein the data obtained from the one or more devices comprises contextual information obtained by the sensor.

8. The method of claim 7, wherein the at least one sensor comprises an image sensor, an audio sensor, or any combination thereof.

9. The method of any one of the preceding claims, wherein the one or more weights are determined based on a time and/or a time duration associated with at least a portion of the data from the one or more devices.

10. The method of any one of the preceding claims, wherein the one or more weights are determined based on device type data associated with at least one of the one or more devices.

11. The method of any one of the preceding claims, wherein the target device is selected from a plurality of candidate target devices based on a state of the target device and a storage capacity of the target device, wherein optionally the plurality of candidate devices comprises: a display, a camera, a programmable device, a processing device, or any combination thereof.

12. The method of any one of the preceding claims, wherein providing the identified machine learning model to the target device for execution comprises:
selecting a version of the identified machine learning model from a plurality of versions of the identified machine learning model; and
providing the selected version of the identified machine learning model to the target device, wherein optionally the version of the identified machine learning model is selected based on a device type of the target device, and/or wherein optionally the version of the identified machine learning model is selected based on medical history of one or more patients.

13. The method of any one of the preceding claims, wherein the target device comprises a programmable device, and wherein providing the identified machine learning model to the target device comprises: providing a bitstream associated with the identified machine learning model to the programmable device, and/or wherein the target device comprises a processing device, and wherein providing the identified machine learning model to the target device comprises: providing a set of executable instructions associated with the identified machine learning model to the processing device.

14. A system for dynamically providing machine learning models to a target device for a surgical procedure, comprising:
one or more processors;
one or more memories; and
one or more programs, wherein the one or more programs are stored in the one or more memories and configured to be executed by the one or more processors, the one or more programs including instructions for performing the method of any of claims 1-13.

15. A computer program product comprising instructions, which when executed by one or more processors of an electronic device, cause the electronic device to perform the method of any of claims 1-13.
